# EUROPEAN PATENT SPECIFICATION

(11) **EP 4 082 580 B1**
(45) Date of publication and mention of the grant of the patent: **10.09.2025**
(21) Application number: 21859651.8
(22) Date of filing: 23.04.2021
(51) Int. Cl.: A61K 51/04, C01B 32/05, A61K 103/32, A61K 51/12, C01B 32/354, C01B 32/372, A61P 35/00

(54) **CARBON MICROBEAD LOADED WITH METAL NUCLIDE, PREPARATION METHOD, AND APPLICATION**
MIT METALLNUKLID BELADENE KOHLENSTOFFMIKROPERLE, HERSTELLUNGSVERFAHREN UND ANWENDUNG
MICROBILLE DE CARBONE CHARGÉE D'UN NUCLÉIDE MÉTALLIQUE, PROCÉDÉ DE PRÉPARATION ET APPLICATION

(30) Priority: 26.08.2020 CN 202010868776
(43) Date of publication of application: 02.11.2022
(73) Proprietor: Chengdu New Radiomedicine Technology Co., Ltd., Chengdu, Sichuan 610200 (CN)
(72) Inventor: ZHAO, Xiaosheng, Ganzhou, Jiangxi 342400 (CN); ZHANG, Chixiang, Chengdu, Sichuan 610036 (CN); LU, Jing, Chongqing 408500 (CN); ZHANG, Jun, Guangyuan, Sichuan 628300 (CN); LI, Zhuoming, Chengdu, Sichuan 610000 (CN); ZHANG, Shuang, Nanchong, Sichuan 637100 (CN); LUO, Xianjin, Zhaotong, Yunnan 657000 (CN); LI, Dongmei, Chengdu, Sichuan 610000 (CN); HU, Xuezheng, Chengdu, Sichuan 610000 (CN); GE, Qiang, Chengdu, Sichuan 610041 (CN); LI, Maoliang, Chengdu, Sichuan 610000 (CN); CAI, Jiming, Chengdu, Sichuan 610041 (CN)
(74) Representative: Ipside
(86) International application number: PCT/CN2021/089201
(87) International publication number: WO 2022/041804

(56) References cited:
- CN-A- 103 771 545
- CN-A- 107 715 122
- CN-A- 107 715 123
- CN-A- 107 715 124
- CN-A- 111 939 276
- US-A1- 2011 038 794
- KIM JUNGHYUN ET AL: "In-situformation of holmium oxide in pores of Mesoporous Carbon Nanoparticles as substrates for neutron-activatable radiotherapeutics", CARBON, ELSEVIER OXFORD, GB, vol. 117, 27 February 2017 (2017-02-27), pages 92 - 99, XP029964681, ISSN: 0008-6223, DOI: 10.1016/J.CARBON.2017.02.085
- LI SHA ET AL: "Preparation and antibacterial property of silver decorated carbon microspheres", APPLIED SURFACE SCIENCE, ELSEVIER, AMSTERDAM , NL, vol. 292, 8 December 2013 (2013-12-08), pages 480 - 487, XP028816302, ISSN: 0169-4332, DOI: 10.1016/J.APSUSC.2013.11.166

## Description

### Technical Field

The present invention relates to the technical fields of chemical industry and pharmaceuticals, in particular to a carbon microbead loaded with a metal nuclide, a preparation method therefor and use thereof.

### Background Art

A carbon microbead is actually a spherical activated carbon, which has the characteristics of high specific surface area, abundant pores, uniform particle size, stable physical and chemical properties, and good biocompatibility, and is widely used in food and pharmaceuticals, energy storage, environmental protection, etc. In order to expand the application of a carbon microbead product in the chemical industry and medical treatment, a metal nuclide is loaded on a carbon microbead.

In the prior art, there are two main methods for loading metal nuclides on carbon microbead products. One is direct adsorption for loading, and the other is using tartaric acid, disodium ethylenediamine tetraacetic acid, etc. for complex adsorption and the loading of carbon microbeads or activated carbon materials is achieved after complexing metal nuclides. The following a), b) articles give examples of direct wet absorption methods for loading carbon microbead products : a) KIM JUNGHYUN ET AL "In-situ formation of holmium oxide in pores of Mesoporous Carbon Nanoparticles as substrates for neutron-activatable radiotherapeutics", CARBON, ELSEVIER OXFORD, GB, vol. 117, 2017-02-27, pages 92-99, b) LI SHA ET AL: "Preparation and antibacterial property of silver decorated carbon microspheres", APPLIED SURFACE SCIENCE, ELSEVIER, AMSTERDAM, NL, vol. 292, 2013-12-08, pages 480-487. The following documents can also be consulted regarding the state of the art of "complex adsorption" for loading of carbon microbeads. Patent application CN 107 715 122 A gives an example of "complex adsorption" using tartaric acid and sodium phosphate, and patent application CN107 715 124 A gives an example using disodium ethylenediamine tetraacetic acid (EDTA). However, in the process of implementation of the inventive technical solutions of the examples of the present application, the inventors of the present application have found that the above-mentioned technologies at least have the following technical problems: Although the direct adsorption method for loading can be used to prepare radioactive granular products, it is only suitable for a few nuclides such as barium - 131, and the loading capacity is small. Furthermore, the radionuclide is partially loaded on the surface of the granules, which requires further encapsulation to prevent the radionuclide from falling off, and the process is complicated. Disodium ethylenediaminetetraacetate complex adsorption method can load radioactive nuclides such as yttrium - 90 into carbon materials, but it usually can't achieve stable loading. The dissolution rate of nuclide in the prepared carbon microbead product loaded with a nuclide increases with the temperature of an aqueous solution; when the temperature of the aqueous solution is higher than 100°C, the dissolution rate or the release rate of the nuclide thereof is more than 2.0%. After 15 minutes of moist heat sterilization at 121°C, it can't meet the indicator requirements on the radioactive release rate of a radioactive microbead product, and there is a great safety risk in clinical use.

### Summary of the Invention

Based on the problems in the background art, an object of the present invention is to provide a carbon microbead loaded with a metal nuclide, a preparation method therefor and use thereof. According to the method of the present invention, a variety of metal nuclides are efficiently loaded on a carbon microbead, and the dissolution rates of the metal nuclides in the prepared carbon microbeads loaded with metal nuclides in an aqueous solution at a temperature lower than 180°C and a pressure lower than 10 MPa are all lower than 0.1%; after 15 minutes of moist heat sterilization at 121°C, the radionuclide release rate thereof is still lower than 0.1%, which can significantly reduce the safety risk of clinical use of radioactive microbead products.

In order to achieve the above object, the technical solution used in the present invention is as follows:
In a first aspect, the present invention provides a method for preparing a carbon microbead loaded with a metal nuclide, the method comprising the following steps:
mixing the metal ions, small organic molecules and water for reaction to obtain a complex;
adsorbing the complex with a carbon microbead; and
performing a first treatment on the adsorbed carbon microbead;
in which, the carbon microbead is a spherical and non-spherical activated carbon product rich in micropores and mesopores; the metal ions in the adsorbed carbon microbead are adsorbed in the pore channels of the carbon microbead.

Further, the small organic molecules are selected from the group consisting of: carboxylic acid group-containing molecules: 5-sulfosalicylic acid, 5-nitrosalicylic acid, trimesic acid, phthalic acid, and isophthalic acid; hydroxy-containing molecules: sodium catechol-3,5-disulfonate, pyrogallol, resorcinol, 8-hydroxyquinoline-5-sulfonic acid; pyridine nitrogen-containing molecules: 2,6-pyridinedicarboxylic acid, 2-pyridinecarboxylic acid, and 1,10-phenanthroline; and/or small molecules with similar structures after simple chemical modification.

Further, a mixed solution of the metal ions, the small organic molecules and water has a pH value of 1.0-12.0, preferably a pH value of 3.0-7.0.

Further, the molar ratio of the small organic molecules to the metal ions is 1-1000 : 1, preferably 1.5-15 : 1.

Further, the metal ions include but are not limited to:
scandium (Sc³⁺), yttrium (Y³⁺), lanthanum (La³⁺), cerium (Ce³⁺), praseodymium (Pr³⁺), neodymium (Nd³⁺), promethium (Pm³⁺), samarium (Sm³⁺), europium (Eu³⁺), gadolinium (Gd³⁺), terbium (Tb³⁺), dysprosium (Dy³⁺), holmium (Ho³⁺), erbium (Er³⁺), thulium (Tm³⁺), ytterbium (Yb³⁺), lutetium (Lu³⁺), copper (Cu²⁺), zinc (Zn²+), iron (Fe³⁺), cobalt (Co³⁺), nickel (Ni³⁺), titanium (Ti⁴⁺), gallium (Ga²⁺), as well as other elements with similar chemical behaviors to the above-mentioned metal elements and all non-radioactive and radioactive isotopes thereof. Different nuclides of the same element, which have the same number of protons and different number of neutrons, are isotopes of each other.

Further, the first treatment is selected from any of the following:
mixing the adsorbed carbon microbead with an anion solution for reaction, and reacting the metal ions adsorbed in the pore channels of the carbon microbead with the anions to form metal salts that are insoluble in water, so as to achieve stable and uniform loading on the carbon microbead;
subjecting the adsorbed carbon microbead to an oxidation treatment, and oxidizing the metal ions adsorbed in the pore channels of the carbon microbead into metal oxides, so as to realize stable loading on the carbon microbead;
subjecting the adsorbed carbon microbead to a reduction treatment, and reducing the metal ions adsorbed in the pore channels of the carbon microbead into metal particles, so as to realize stable loading on the carbon microbead.

Further, the anion solution includes, but is not limited to, any one of phosphate, carbonate, tannin, and sulfide ion solutions.

Further, the oxidation treatment is a high-temperature treatment in an inert gas atmosphere or an atmosphere containing trace oxygen.

Further, the reduction treatment is a treatment with a reducing agent or a high-temperature reducing gas atmosphere.

In a second aspect, the present invention provides a carbon microbead loaded with a metal nuclide, which is prepared by the method of the first aspect, wherein the dissolution rate of the metal nuclide in the carbon microbead loaded with a metal nuclide in the aqueous solution at a temperature lower than 180°C and a pressure lower than 10 MPa is lower than 0.1%.

In a third aspect, the present invention provides a carbon microbead loaded with a metal nuclide, wherein the carbon microbead loaded with a metal nuclide of the second aspect is used for but not limited to medical uses.

Further, the particle size and shape of the carbon microbead product will not affect the implementation and application of the method.

Further, when used for medical uses (for example, preparing a medicine for the treatment of tumor embolization with radioactive internal irradiation), the carbon microbead product has a particle size of 0.1-1000 µm.

Further, when used as a radioactive carbon microbead product for the treatment of solid liver tumor embolization with radioactive internal irradiation, the carbon microbead loaded with a metal nuclide has an optimal particle size of 20-60 µm.

Compared with the prior art, the beneficial effects of the present invention are:
The preparation method of the present invention can simultaneously realize efficient, stable and uniform loading of single or multiple metal ions on the carbon microbead, and these metal ions are stably and uniformly distributed in the pores of a carbon material. All the prepared carbon microbeads loaded with metal nuclides can exist stably in an aqueous solution at a temperature lower than 180°C and a pressure lower than 10 MPa, and the dissolution rates of the metal nuclides in the aqueous solution are all lower than 0.1%; after 15 minutes of moist heat sterilization at 121°C, the radionuclide release rate thereof is still lower than 0.1%, which can significantly reduce the safety risk of clinical use of radioactive microbead products.

Only small organic molecules are introduced into the product of the method of the present invention as impurities and the amount is controllable. The carbon microbead product for medicine uses can be selected from small organic molecules with good biocompatibility. The carbon microbead loaded with a metal nuclide product prepared by the method can be used for medicine uses.

The method of the present invention has a simple preparation process, can be implemented quickly, and is easy to realize industrialized production.

### Brief Description of the Drawings

Fig. 1 is the diagram of yttrium loading rate of the carbon microbead vs liquid-solid ratio in the example 11 of the present invention;
Fig. 2 is the diagram of yttrium loading rate of the carbon microbead vs the molar ratio of 5-sulfosalicylic acid to yttrium in the example 11 of the present invention;
Fig. 3 is the diagram of yttrium loading rate of the carbon microbead vs the pH value of the complexing solution in the example 11 of the present invention;
Fig. 4 is the diagram of yttrium loading rate of the carbon microbead vs adsorption temperature (T) in the example 11 of the present invention;
Fig. 5 is the diagram of yttrium loading rate of the carbon microbead vs adsorption time (t) in the example 11 of the present invention;
Fig. 6 is the diagram of yttrium loading rate of the carbon microbead vs the addition amount of yttrium in the example 11 of the present invention.

### Detailed Description of Examples

Specific examples of the present invention will be further described below in conjunction with the drawings. It should be noted here that the description of the examples is intended to help understand the present invention, but does not constitute a limitation to the present invention. In addition, the technical features involved in the examples of the present invention as described below can be combined with each other so long as they do not constitute a conflict with each other.

### Example 1

A method for preparing a carbon microbead loaded with a metal nuclide, the method comprising the following steps:
mixing the metal ions, small organic molecules and water for reaction to obtain a complex; specifically, small organic molecules include, 5-sulfosalicylic acid, 5-nitrosalicylic acid, trimesic acid, phthalic acid, and isophthalic acid; sodium catechol-3,5-disulfonate, pyrogallol, resorcinol, 8-hydroxyquinoline-5-sulfonic acid; 2,6-pyridinedicarboxylic acid, 2-pyridinecarboxylic acid, 1,10-phenanthroline. These small organic molecules are easy to form complexes with metal ions in an aqueous solution with a certain pH value;
adsorbing the complex with a carbon microbead; specifically, spherical and non-spherical activated carbon rich in micropores and mesopores are used to adsorb the complexes formed by metal ions and small organic molecules in an aqueous solution. Under the above pH value conditions, a carbon microbead can rapidly and efficiently adsorb these complexes into their pore channels to achieve uniform loading; and
performing a first treatment on the adsorbed carbon microbead.

### Example 2

Further, a pH value suitable for the complex reaction of the small organic molecules and the metal ions in an aqueous solution is 1.0-12.0, and preferably, the pH value is 3.0-7.0.

### Example 3

Further, the molar ratio of the small organic molecules to the metal ions is 1-1000 : 1, preferably 1.5-15 : 1.

### Example 4

Further, the metal ions include:
scandium (Sc³⁺), yttrium (Y³⁺), lanthanum (La³⁺), cerium (Ce³⁺), praseodymium (Pr³⁺), neodymium (Nd³⁺), promethium (Pm³⁺), samarium (Sm³⁺), europium (Eu³⁺), gadolinium (Gd³⁺), terbium (Tb³⁺), dysprosium (Dy³⁺), holmium (Ho³⁺), erbium (Er³⁺), thulium (Tm³⁺), ytterbium (Yb³⁺), lutetium (Lu³⁺), copper (Cu²⁺), zinc (Zn²⁺), iron (Fe³⁺), cobalt (Co³⁺), nickel (Ni³⁺), titanium (Ti⁴⁺), gallium (Ga²⁺)and all non-radioactive and radioactive isotopes thereof.

In which, "isotopes" refer to that different nuclides of the same element, which have the same number of protons and different number of neutrons, are isotopes of each other.

### Example 5

Furthermore, the first treatment comprises mixing the adsorbed carbon microbead with an anion solution for reaction, and reacting the metal ions adsorbed in the pore channels of the carbon microbead with the anions to form metal salts that are insoluble in water, so as to achieve stable and uniform loading on the carbon microbead;
wherein the anion solution includes, any one of phosphate, carbonate, tannin, and sulfide ion solutions.

### Example 6

Furthermore, the first treatment comprises subjecting the adsorbed carbon microbead to an oxidation treatment, and oxidizing the metal ions adsorbed in the pore channels of the carbon microbead into metal oxides, so as to realize stable loading on the carbon microbead; wherein the oxidation treatment is a high-temperature treatment in an inert gas atmosphere or an atmosphere containing trace oxygen.

### Example 7

Furthermore, the first treatment comprises subjecting the adsorbed carbon microbead to a reduction treatment, and reducing the metal ions adsorbed in the pore channels of the carbon microbead into metal particles, so as to realize stable loading on the carbon microbead; wherein the reduction treatment is a treatment with a reducing agent or a high-temperature reducing gas atmosphere.

### Example 8

A carbon microbead loaded with a metal nuclide, which is prepared by the method of any of the preceding examples, wherein the dissolution rates of the metal nuclides in the carbon microbeads loaded with metal nuclides in an aqueous solution at a temperature lower than 180°C and a pressure lower than 10 MPa are all lower than 0.1%.

### Example 9

The method for preparing a carbon microbead loaded with a metal nuclide of the above examples can also be used to prepare a metal-doped carbon material, wherein the prepared carbon microbead loaded with a metal nuclide is used for but not limited to medical uses.

### Example 10

Furthermore, the carbon microbead loaded with a metal nuclide is used for medical uses, wherein the particle size of the carbon microbead is 0.1-1000 µm; preferably, when used as a radioactive carbon microbead product for the treatment of the solid liver tumor embolization with radioactive internal irradiation, the carbon microbead loaded with a metal nuclide has an optimal particle size of 20-60 µm.

### Example 11

There are many factors that affect the loading of metal ions on the carbon microbead, and the chemical behaviors of rare earth elements are very similar. In this example, taking yttrium as a representative, the effects of liquid-solid ratio, molar ratio, pH value, adsorption time, adsorption temperature and the addition amount of metal ions, etc., on the loading behavior of rare earth nuclide ions on the carbon microbead are studied.

### 11.1 Preparation method of carbon microbead loaded with yttrium

Mixing yttrium ions, small organic molecules and water for reaction to obtain a complex, and the specific operation is as follows:
taking a certain volume of 1.0 g/L yttrium chloride aqueous solution, placing it in a reaction flask, and then adding a certain volume of 1.0 g/L 5-sulfosalicylic acid solution, such that the molar weight of 5-sulfosalicylic acid is several times of that of yttrium ions, adding a certain volume of water to adjust the pH value of the solution, shaking same well to obtain a complex for later use;
adsorbing the complex with a carbon microbead, and the specific operation is as follows:
   weighing 0.25 g of the carbon microbead with a particle size of 20-60 µm and adding it to the above complex solution;
   performing a first treatment on the adsorbed carbon microbead, and the specific operation is as follows:
      adding a certain volume of sodium phosphate solution to the above adsorbed carbon microbead solution, and shaking same for 1 minute to obtain a carbon microbead loaded with yttrium.

### 11.2 Effect of different conditions on the yttrium ion loading rate

The carbon microbeads are added to the complex solution and then shaken for a certain time at a certain temperature, and the supernatant is taken to measure the yttrium content to obtain the loading rate of yttrium ions on the carbon microbead. The effects of different liquid-solid ratios, different molar ratios, different pH values, different temperatures, different times, and different yttrium addition amounts on the adsorption of yttrium ions by carbon microbeads are studied respectively.

In which, the liquid-solid ratio is the ratio of the solution mass (g) to the carbon microbead mass (g) during adsorption; the molar ratio is the molar ratio of small organic molecule 5-sulfosalicylic acid to yttrium; the pH value is the pH value of the mixed solution of the metal ions, the small organic molecules and water; the temperature is the temperature of the reaction system aqueous solution; and the time is the time for the carbon microbead to adsorb yttrium.

### (1) Effect of different liquid-solid ratios

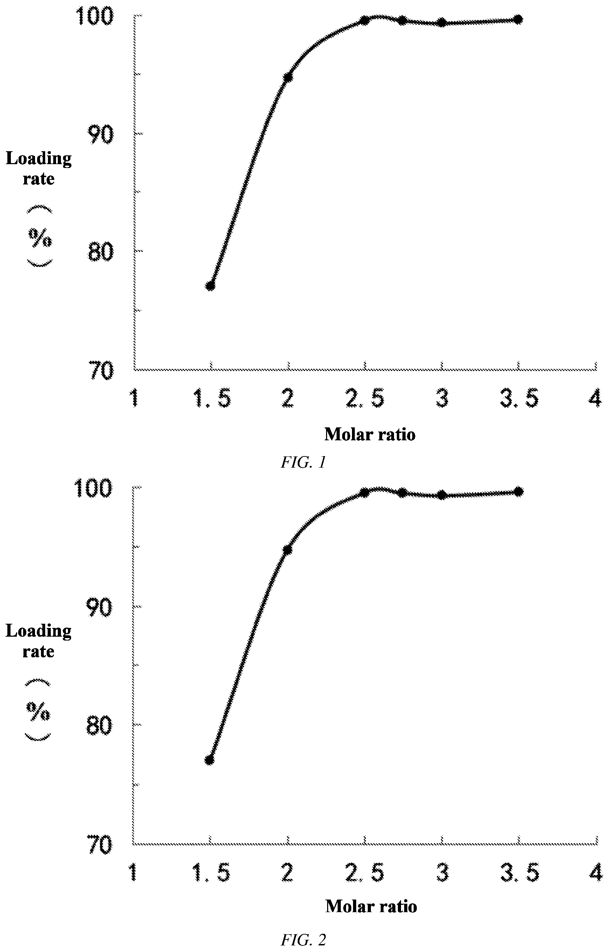

In this example, the carbon microbeads loaded with yttrium prepared with the following liquid-solid ratios are selected respectively for tests: 20 : 1, 50 : 1, 100 : 1, 200 : 1, 300 : 1, 400 : 1, and 500 : 1, and the test results are shown in Fig. 1.

It can be seen from Fig. 1 that when the liquid-solid ratio is less than 100 times, the loading rate of yttrium by the carbon microbead can be maintained above 98%. Therefore, the preferred liquid-solid ratio is less than or equal to 100 times.

### (2) Effect of different molar ratios

In this example, the carbon microbeads loaded with yttrium prepared with the following molar ratios are selected respectively for tests: 1.5 : 1, 2 : 1, 2.5 : 1, 2.7 : 1, 3 : 1, and 3.5 : 1, and the test results are shown in Fig. 2.

It can be seen from Fig. 2 that when the molar ratio of 5-sulfosalicylic acid to yttrium is greater than 2.5, the loading rate of yttrium by the carbon microbead can be maintained above 98%, and the preferred molar ratio is greater than or equal to 2.5.

### (3) Effect of different pH values

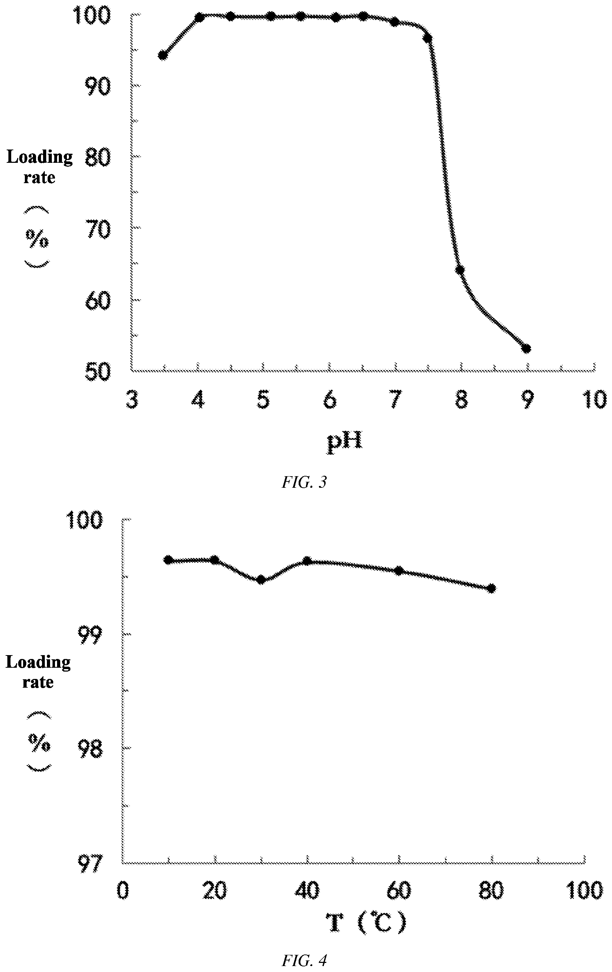

In this example, the carbon microbeads loaded with yttrium prepared with the following pH values are selected respectively for tests: 3.5, 4.0, 4.5, 5.0, 5.5, 6.0, 6.5, 7.0, 7.5, 8.0, and 9.0, and the test results are shown in Fig. 3.

It can be seen from Fig. 3 that when the pH value is 4.0-7.0, the loading rate of yttrium by the carbon microbead can be maintained above 98%, and the preferred pH value is 4.0-7.0.

### (4) Effect of different temperatures

In this example, the carbon microbeads loaded with yttrium prepared at the following temperatures of the reaction system aqueous solutions are selected respectively for tests: 10, 20, 30, 40, 60, and 80°C, and the test results are shown in Fig. 4.

It can be seen from Fig. 4 that the temperature has little influence on the adsorption of yttrium by the carbon microbead, and efficient loading can be achieved within the temperature range in the examples of the present invention.

### (5) Effect of different times

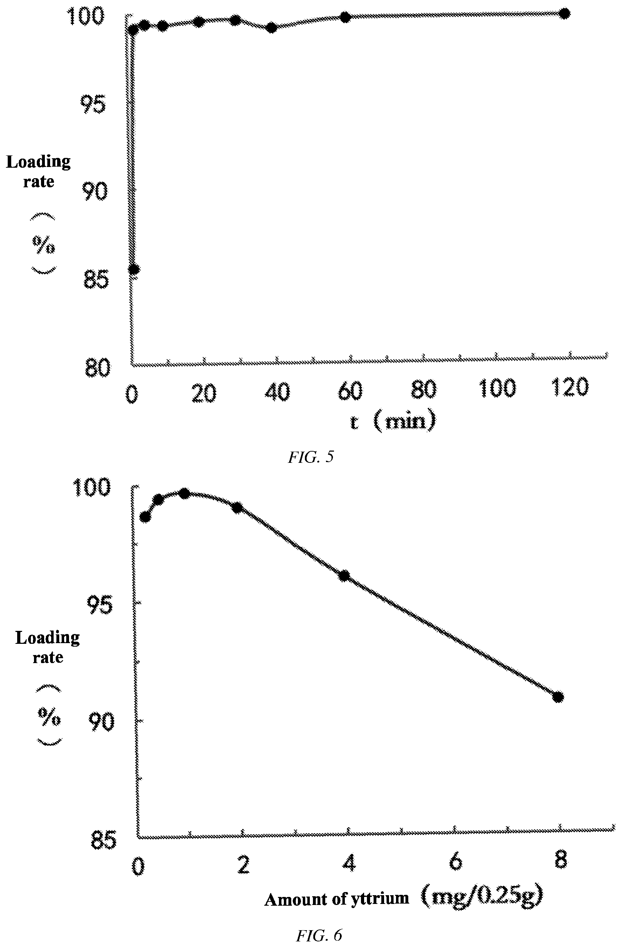

In this example, the carbon microbeads loaded with yttrium prepared with the following adsorption time are selected respectively for tests: 1, 2, 5, 10, 20, 30, 40, 60, and 120 min, and the test results are shown in Fig. 5.

It can be seen from Fig. 5 that the adsorption of yttrium by the carbon microbead is a fast kinetic process, and the loading rate has been greater than 99% when it takes more than or equal to 2 minutes.

### (6) Effect of different addition amounts of yttrium

In this example, the carbon microbeads loaded with yttrium prepared with the following addition amounts of yttrium are selected respectively for tests: 0.2, 0.5, 1, 2, 4, and 8 mg/0.25 g, and the test results are shown in Fig. 6.

It can be seen from Fig. 6 that the addition amount of yttrium has a certain influence on the adsorption rate. When the amount of yttrium increases to 32 mg/g, the loading rate of yttrium adsorbed by the carbon microbead is 90.7%, indicating that the saturated loading capacity of yttrium per gram of carbon microbeads can be greater than 32 mg.

It should be noted that: the adsorption behavior of the carbon microbead on other rare earth elements is very similar to that of the carbon microbead on yttrium, and there is no significant difference. The data of the influencing factor research tests will not be provided in detail in the subsequent examples.

### Example 12

### Preparation of medical carbon microbeads loaded with yttrium-90

Mixing yttrium-90 ions, small organic molecules and water for reaction to obtain a complex, and the specific operation is as follows: taking 1 ml of 1.0 g/L yttrium trichloride aqueous solution and adding it in a reaction flask, adding a radioactive yttrium-90 solution with an activity of 2 curies, and then adding a certain volume of 1.0 g/L 5-sulfosalicylic acid solution, such that the molar weight of 5-sulfosalicylic acid is 5 times of that of yttrium, adding water to the scale of 50 ml, so as to adjust the pH value of the solution to 4.5, then adding water with a pH value of 4.5 to 100 ml, shaking same well to obtain a complex for later use;
adsorbing the complex with a carbon microbead, and the specific operation is as follows: weighing 1.0 g of the carbon microbead with a particle size of 20-60 µm and adding same to the above complex solution, and stirring same for 20 minutes to complete the adsorption;
performing a first treatment on the adsorbed carbon microbead, and the specific operation is as follows: taking the supernatant to measure the radioactivity activity of yttrium-90, and the calculated adsorption rate of the radionuclide is 99.2%; adding 150 ml of 0.1 mol/L sodium phosphate solution to the adsorbed carbon microbead solution, and stirring same for 5 minute to obtain the product.

Cleaning and subpacking of yttrium-90 carbon microbead: washing the yttrium-90 carbon microbead product twice with water for injection, after solid-liquid separation, the yttrium-90 carbon microbead solid is obtained; then adding 10 ml of water for injection and dividing the product into 10 bottles evenly, wherein the product in each bottle has a volume of 1 ml and a radioactivity activity of about 195 mCi/ bottle, and each bottle contains about 100 mg of yttrium-90 carbon microbead. After 15 minutes of sterilization at 121°C, a yttrium -90 carbon microbead product for injection is obtained.

Determination of the dissolution rate of yttrium-90 nuclide in yttrium-90 carbon microbead: taking about 20 mg of the above yttrium-90 carbon microbeads, respectively, and placing same in a hydrothermal reaction kettle, adding 20 ml of water for injection or 0.9% sodium chloride solution, sealing same and placing in a constant temperature oven, and setting the temperature-raising and constant-temperature programs to start running; after cooling to room temperature, centrifuging same to take a certain volume of supernatant for activity measurement and calculating the dissolution rate. The release rate of yttrium-90 nuclide at a constant temperature of 40°C (1.0 MPa), 80°C (1.0 MPa), 100°C (1.0 MPa), 120°C (2.0 MPa), 140°C (3.6 MPa), 160°C (6.1 MPa), and 180°C (10.0 MPa) for 2 hours are investigated, respectively. The results are shown in table 1 below:

**Table 1. Dissolution rates of yttrium-90 in yttrium-90 carbon microbeads in aqueous solutions at different temperatures and pressures**

| No. | Experimental conditions (temperature; pressure; time) | Dissolution rate of yttrium-90 (0.9% sodium chloride solution) | Dissolution rate of yttrium-90 (water for injection) |
|---|---|---|---|
| 1 | 40°C; 1.0 MPa; 2h | 0.0732% | 0.0094% |
| 2 | 80°C; 1.0 MPa; 2h | 0.0718% | 0.0334% |
| 3 | 100°C; 1.0 MPa; 2h | 0.0429% | 0.0383% |
| 4 | 120°C; 2.0 MPa; 2h | 0.0216% | 0.0323% |
| 5 | 140°C; 3.6 MPa; 2h | 0.0782% | 0.0158% |
| 6 | 160°C; 6.1 MPa; 2h | 0.0656% | 0.0718% |
| 7 | 180°C; 10.0 MPa; 2h | 0.0753% | 0.0824% |

The results in table 1 show that the dissolution rates of the yttrium-90 nuclides in the yttrium-90 carbon microbead in water for injection and 0.9% sodium chloride solution at 40-180°C (a pressure of 1.0-10.0 MPa) are all lower than 0.1%.

### Example 13

### Preparation of carbon microbead loaded with yttrium-90

Mixing yttrium-90, small organic molecules and water for reaction to obtain a complex, and the specific operation is as follows: taking of 1 ml of 1.0 g/L yttrium trichloride aqueous solution and adding same in a reaction flask, adding a radioactive yttrium-90 solution with an activity of 10 mCi, and then adding a certain volume of 1.0 g/L 5-nitrosalicylic acid solution, such that the molar weight of 5-nitrosalicylic acid is 6 times of that of yttrium, adding water to the scale of 50 ml, so as to adjust the pH value of the solution to 5.5, then adding water with a pH value of 5.5 to 100 ml, and shaking same well to obtain a complex for later use;
adsorbing the complex with a carbon microbead, and the specific operation is as follows: weighing 1.0 g of the carbon microbead with a particle size of 20-60 µm and adding same to the above complex solution, and stirring same for 20 minutes to complete the adsorption;
performing a first treatment on the adsorbed carbon microbead, and the specific operation is as follows: taking the supernatant to measure the radioactivity activity of the yttrium-90, and the calculated adsorption rate of the radionuclide is 98.1%. Adding 150 ml of 0.1 mol/L sodium phosphate solution to the adsorbed carbon microbead solution, and stirring same for 5 minute to obtain the product.

Cleaning, sterilization and subpacking of yttrium-90 carbon microbeads: washing the yttrium-90 carbon microbead product twice with water for injection, after solid-liquid separation, the yttrium-90 carbon microbead solid is obtained; then adding 10 ml of water for injection and dividing the product into 10 bottles evenly, wherein the product in each bottle has a volume of 1 ml and a radioactivity activity of about 0.96 mCi/ bottle, and each bottle contains about 100 mg of yttrium-90 carbon microbeads. After 15 minutes of sterilization at 121°C, the product is obtained.

Determination of the dissolution rate of yttrium-90 in yttrium-90 carbon microbead: taking a bottle of the above product, centrifuging and taking the supernatant for an activity test, the calculated dissolution rate of yttrium-90 is 0.0435%.

### Example 14

### Preparation of medical carbon microbead loaded with lutecium-177

Mixing lutecium-177, small organic molecules and water for reaction to obtain a complex, and the specific operation is as follows: taking 0.2 ml of 1.0 g/L lutecium trichloride aqueous solution and adding same in a reaction flask, adding a radioactive lutecium-177 solution with an activity of 0.8 Ci, and then adding a certain volume of 1.0 g/L 5-sulfosalicylic acid solution, such that the molar weight of 5-sulfosalicylic acid is 4 times of that of lutecium, adding water to the scale of 20 ml, so as to adjust the pH value of the solution to 5.0, then adding water with a pH value of 5.0 to 30 ml, and shaking same well to obtain a complex for later use;
adsorbing the complex with a carbon microbead, and the specific operation is as follows: weighing 0.3 g of the carbon microbead with a particle size of 20-60 µm and adding same to the above complex solution, and stirring same for 20 minutes to complete the adsorption;
performing a first treatment on the adsorbed carbon microbead, and the specific operation is as follows: taking the supernatant to measure the radioactivity activity of the lutecium-177, and the calculated adsorption rate of the radionuclide is 99.3%. Adding 150 ml of 0.1 mol/L sodium phosphate solution to the adsorbed carbon microbead solution, and stirring same for 5 minutes.

Cleaning and subpacking of lutecium-177 carbon microbead: washing the lutecium-177 carbon microbead product twice with water for injection, after solid-liquid separation, the lutecium-177 carbon microbead solid is obtained; then adding 6 ml of water for injection and dividing the product into 3 bottles evenly, wherein the product in each bottle has a volume of 2 ml and a radioactivity activity of about 250 mCi/ bottle, and each bottle contains about 100 mg of lutecium-177 carbon microbead. After 15 minutes of sterilization at 121°C, a lutecium-177 carbon microbead product for injection is obtained.

Determination of the dissolution rate of lutecium-177 nuclide in lutecium-177 carbon microbead: taking 20 mg of the above lutecium-177 carbon microbeads, respectively, and placing same in a hydrothermal reaction kettle, adding 20 ml of water for injection or 0.9% sodium chloride solution, sealing them and placing in a constant temperature oven, keeping the temperature constant at 120°C for 2h; after cooling to room temperature, centrifuging same to take a certain volume of supernatant for activity measurement and calculating the dissolution rate. The results show that the dissolution rate of lutetium-177 carbon microbeads in 0.9% sodium chloride solution is 0.0788%, and the dissolution rate in water for injection is 0.0548%.

### Example 15

### Preparation of medical holmium-166/holmium-165 carbon microbead

Mixing holmium-166/holmium-165, small organic molecules and water for reaction to obtain a complex, and the specific operation is as follows: taking 0.5 ml of 0.5 g/L holmium chloride (holmium-165) aqueous solution and adding same in a reaction flask, adding a radioactive holmium-166 solution with an activity of 0.5 curies, and then adding a certain volume of 1.0 g/L 5-sulfosalicylic acid solution, such that the molar weight of 5-sulfosalicylic acid is 4.5 times of that of holmium, adding water to the scale of 30 ml, so as to adjust the pH value of the solution to 4.2, and shaking same well to obtain a complex for later use;
adsorbing the complex with a carbon microbead, and the specific operation is as follows: weighing 0.3 g of the carbon microbead with a particle size of 20-60 µm and adding same to the above complex solution, and stirring same for 10 minutes to complete the adsorption;
performing a first treatment on the adsorbed carbon microbead, and the specific operation is as follows: taking the supernatant to measure the radioactivity activity of holmium-166, and the calculated adsorption rate of the radionuclide is 98.6%. Adding 150 ml of 0.1 mol/L sodium phosphate solution to the adsorbed carbon microbead solution, and stirring same for 5 minutes.

Cleaning and subpacking of holmium carbon microbead: washing the holmium carbon microbead product twice with 30 ml of water for injection, after solid-liquid separation, the holmium carbon microbead solid is obtained; then adding 6 ml of water for injection and dividing the product into 3 bottles evenly, wherein the product in each bottle has a volume of 2 ml and a radioactivity activity of about 120 mCi/ bottle, and each bottle contains about 100 mg of holmium carbon microbead. After 15 minutes of sterilization at 121°C, a holmium-166/holmium-165 carbon microbead product for injection is obtained.

Determination of the dissolution rate of holmium-166 nuclide in holmium-166/holmium-165 carbon microbead: taking 10 mg of the above holmium-166/holmium-165 carbon microbeads, respectively, and placing same in a hydrothermal reaction kettle, adding 10 ml of water for injection or 0.9% sodium chloride solution, sealing same and placing in a constant temperature oven, keeping the temperature constant at 120°C for 2 h; after cooling to room temperature, centrifuging same to take a certain volume of supernatant for activity measurement and calculating the dissolution rate. The results show that the dissolution rate of holmium-166 carbon microbeads in 0.9% sodium chloride solution is 0.0188%, and the dissolution rate in water for injection is 0.0245%.

### Example 16

### Preparation of medical carbon microbead loaded with samarium-153

Mixing samarium-153, small organic molecules and water for reaction to obtain a complex, and the specific operation is as follows: taking 1 ml of 0.5 g/L samarium trichloride aqueous solution and adding same in a reaction flask, adding a radioactive samarium-153 solution with an activity of 0.82 curies, and then adding a certain volume of 1.0 g/L 5-sulfosalicylic acid solution, such that the molar weight of 5-sulfosalicylic acid is 4 times of that of samarium, adding water to the scale of 30 ml, so as to adjust the pH value of the solution to 5.6, then adding water with a pH value of 5.6 to 50 ml, and shaking same well to obtain a complex for later use;
adsorbing the complex with a carbon microbead, and the specific operation is as follows: weighing 0.5 g of the carbon microbead with a particle size of 20-60 µm and adding same to the above complex solution, and stirring same for 10 minutes to complete the adsorption;
performing a first treatment on the adsorbed carbon microbead, and the specific operation is as follows: taking the supernatant to measure the radioactivity activity of samarium-153, and the calculated adsorption rate of the radionuclide is 99.3%. Adding 50 ml of 0.1 mol/L sodium phosphate solution to the adsorbed carbon microbead solution, and stirring same for 5 minute to obtain the product.

Cleaning and subpacking of samarium-153 carbon microbead: washing the samarium-153 carbon microbead product twice with 50 ml of water for injection, after solid-liquid separation, the samarium-153 carbon microbead solid is obtained; then adding 10 ml of water for injection and dividing the product into 5 bottles evenly, wherein the product in each bottle has a volume of 1 ml and a radioactivity activity of about 150 mCi/ bottle, and each bottle contains about 100 mg of samarium-153 carbon microbead. After 15 minutes of sterilization at 121°C, a samarium-153 carbon microbead product for injection is obtained.

Determination of the dissolution rate of samarium-153 nuclide in the carbon microbead loaded with samarium-153: taking 10 mg of the above samarium-153 carbon microbeads, respectively, and placing same in a hydrothermal reaction kettle, adding 10 ml of water for injection or 0.9% sodium chloride solution, sealing same and placing in a constant temperature oven, keeping the temperature constant at 120°C for 2 h; after cooling to room temperature, centrifuging same to take a certain volume of supernatant for activity measurement and calculating the dissolution rate. The results show that the dissolution rate of samarium-153 carbon microbeads in 0.9% sodium chloride solution is 0.0326%, and the dissolution rate in water for injection is 0.0137%.

### Example 17

Preparation of carbon microbeads loaded with light rare earths (lanthanum, cerium, praseodymium, neodymium, promethium, samarium and europium)

Mixing light rare earth ions, small organic molecules and water for reaction to obtain a complex, and the specific operation is as follows: taking 3 ml of 0.5 g/L aqueous solutions of lanthanum chloride, cerium chloride, praseodymium chloride, neodymium chloride, promethium chloride, samarium chloride and europium chloride, respectively, adding same into a reaction flask, shaking same well, and then adding a certain volume of 1.0 g/L 5-sulfosalicylic acid solution, such that the molar weight of 5-sulfosalicylic acid is 8 times of that of rare earth ions, adjusting the pH value of the solution to 4.8, and shaking same well to obtain a complex for later use;
adsorbing the complex with a carbon microbead, and the specific operation is as follows: weighing 3.0 g of the carbon microbead and adding same to the above complex solution, and stirring same for 10 minutes to complete the adsorption;
performing a first treatment on the adsorbed carbon microbead, and the specific operation is as follows: taking the supernatant and using an inductively coupled atomic emission spectrometer to determine the content of lanthanum, cerium, praseodymium, neodymium, promethium, samarium and europium, and the calculated average loading rate of lanthanum, cerium, praseodymium, neodymium, promethium, samarium and europium is 98.3%. The first treatment method includes:
   Method 1: taking 200 mg of the above carbon microbeads loaded with light rare earth nuclides, adding 50 ml of 0.1 mol/L sodium carbonate aqueous solution, shaking same well for reaction for 10 minutes, after solid-liquid separation, drying same to obtain a solid. According to this method, the rare earth nuclides in the carbon microbeads doped with rare earth mainly exist in the carbon microbeads in the form of carbonate.
   Method 2: taking 200 mg of the above carbon microbeads loaded with light rare earth nuclides, adding 50 ml of 0.1 mol/L sodium phosphate aqueous solution, and stirring same for 5 minutes. According to this method, the rare earth nuclides in the carbon microbeads doped with rare earth mainly exist in the form of phosphate.
   Method 3: taking 200 mg of the above carbon microbeads loaded with light rare earth nuclides, adding 50 ml of 0.1 mol/L tannin solution, and stirring same for 5 minutes.
   Method 4: taking 200 mg of the above carbon microbeads loaded with light rare earth nuclides, placing same in a high-temperature furnace, keeping the temperature constant at 800°C for 2 hours, and washing them with water to neutrality after cooling. In this case, rare earth nuclides are mainly loaded in the carbon microbeads in the form of rare earth oxides.
   Method 5: taking 200 mg of the above carbon microbeads loaded with light rare earth nuclides, placing same in a high-temperature furnace under hydrogen atmosphere, keeping the temperature constant at 800°C for 2 hours, and washing them with water to neutrality after cooling. In this case, rare earth nuclides are mainly loaded in the carbon microbeads in the form of rare earth metals.
   Method 6: taking 200 mg of the above carbon microbeads loaded with light rare earth nuclides, placing same in a high-temperature furnace under the atmosphere of hydrogen sulfide and carbon disulfide, keeping the temperature constant at 900°C for 3 hours, and washing them with water to neutrality after cooling. In this case, rare earth nuclides are mainly loaded in the carbon microbeads in the form of rare earth sulfides.

The chemical behavior of rare earth nuclide ions is similar. The above 6 curing methods are also suitable for the curing of other rare earth nuclides on carbon microbead.

Determination of the dissolution rate of light rare earth nuclides in the carbon microbeads loaded with light rare earth: taking about 50 mg of carbon microbead products obtained in the above methods 1-6, respectively, and placing same in a hydrothermal reaction kettle, adding 10 ml of purified water, sealing same and placing in a constant temperature oven, and keeping the temperature constant at 120°C for 2 h; after cooling to room temperature, centrifuging same to take a certain volume of supernatant for the content test of light rare earth nuclide ions. The results show that the total dissolution rates of light rare earth nuclides in water in methods 1-6 are 0.065%, 0.096%, 0.071%, 0.068%, 0.091% and 0.077%, respectively.

### Example 18

Preparation of carbon microbeads loaded with heavy rare earths (gadolinium, terbium, dysprosium, holmium, erbium, thulium, ytterbium, lutetium, yttrium)

Mixing heavy rare earth ions, small organic molecules and water for reaction to obtain a complex, and the specific operation is as follows: taking 2 ml of 0.5 g/L aqueous solutions of gadolinium chloride, terbium chloride, dysprosium chloride, holmium chloride, erbium chloride, thulium chloride, ytterbium chloride, lutecium chloride and yttrium chloride, respectively, and adding same into a reaction flask, shaking same well, then adding a certain volume of 1.0 g/L 5-sulfosalicylic acid solution, such that the molar weight of 5-sulfosalicylic acid is 10 times of that of rare earth ions, adjusting the pH value of the solution to 4.9, then adding water with a pH value of 4.9 to 200 ml, and shaking same well to obtain a complex for later use;
adsorbing the complex with a carbon microbead, and the specific operation is as follows: weighing 5.0 g of the carbon microbead and adding same to the above solution, and stirring for 10 minutes to complete the adsorption;
performing a first treatment on the adsorbed carbon microbead, and the specific operation is as follows: taking the supernatant and using an inductively coupled atomic emission spectrometer to determine the content of gadolinium, terbium, dysprosium, holmium, erbium, thulium, ytterbium, lutetium and yttrium, and the calculated average loading rate is 98.8%; adding 150 ml of 0.1 mol/L sodium phosphate solution to the above carbon microbeads loaded with heavy rare earth nuclides, shaking same for 5 min, washing the carbon microbeads twice with purified water, followed by solid-liquid separation.

Determination of the dissolution rate of heavy rare earth nuclides in the carbon microbeads loaded with heavy rare earth: taking about 50 mg of the above microbead products, respectively, and placing same in a hydrothermal reaction kettle, adding 10 ml of purified water, sealing same and placing in a constant temperature oven, and keeping the temperature constant at 120°C for 2 h; after cooling to room temperature, centrifuging same to take a certain volume of supernatant for the content test of heavy rare earth nuclide ions. The results show that the total dissolution rate of heavy rare earth nuclides in water is 0.087%.

### Example 19

### Preparation of carbon microbead loaded with iron

Mixing iron ions, small organic molecules and water for reaction to obtain a complex, and the specific operation is as follows: taking 10 ml of 0.5 g/L iron chloride aqueous solution and adding same into a reaction flask, and then adding a certain volume of 0.5 g/L sodium catechol-3,5-disulfonate solution, such that the molar weight of sodium catechol-3,5-disulfonate is 2 times of that of iron ions, adjusting the pH value of the solution to 3.5, and shaking same well to obtain a complex for later use;
adsorbing the complex with a carbon microbead, and the specific operation is as follows: weighing 1.0 g of the carbon microbead and adding same to the above solution, and stirring same for 10 minutes to complete the adsorption;
performing a first treatment on the adsorbed carbon microbead, and the specific operation is as follows: taking the supernatant to measure the iron content by the spectrophotometry method, and the calculated loading rate is 99.3%; taking the above carbon microbeads loaded with iron, placing same in a high-temperature furnace after drying, keeping the temperature constant at 600°C for 2 hours, and washing same with purified water to neutrality after cooling to obtain the carbon microbead loaded with iron oxide.

The chemical properties of cobalt and nickel are similar to those of iron, and a carbon microbead loaded with nickel and a carbon microbead loaded with cobalt can also be prepared by this method.

Determination of the dissolution rate of iron ions in the carbon microbead loaded with iron: taking about 100 mg of the above microbead s, and placing same in a hydrothermal reaction kettle, adding 10 ml of purified water, sealing and placing same in a constant temperature oven, and keeping the temperature constant at 120°C for 2 h; after cooling to room temperature, centrifuging same to take a certain volume of supernatant for the content test of iron ions. The results show that the dissolution rate of iron in water is 0.098%.

### Example 20

### Preparation of carbon microbead loaded with copper

Mixing copper ions, small organic molecules and water for reaction to obtain a complex, and the specific operation is as follows: taking 10 ml of 0.1 g/L copper chloride aqueous solution and adding same into a reaction flask, then adding a certain volume of 1.0 g/L 2,6-pyridinedicarboxylic acid solution, such that the molar weight of 2,6-pyridinedicarboxylic acid is 2.5 times of that of iron ions, adjusting the pH value of the solution to 5.5, shaking same well to obtain a complex for later use;
adsorbing the complex with a carbon microbead, and the specific operation is as follows: weighing 1.0 g of the carbon microbead and adding same to the above solution, and stirring same for 10 minutes to complete the adsorption;
performing a first treatment on the adsorbed carbon microbead, and the specific operation is as follows: taking the supernatant to measure the copper content by the spectrophotometry method, and the calculated loading rate is 98.4%. The first treatment comprises the following methods:
   Method 1: taking 200 mg of the above carbon microbead loaded with copper, adding same to 0.1 mol/L sodium sulfide solution, shaking same for 2 min, after solid-liquid separation, drying same to obtain a carbon microbead loaded with copper sulphide.
   Method 2: taking 200 mg of the above carbon microbeads loaded with copper, carrying out solid-liquid separation, placing same in a high-temperature furnace after drying, and keeping the temperature constant at 600°C for 2 hours to obtain a carbon microbead loaded with copper oxide.

Determination of the dissolution rate of copper ions in the carbon microbead loaded with copper: taking about 100 mg of the carbon microbead products from the above methods 1 and 2, respectively, and placing same in a hydrothermal reaction kettle, adding 10 ml of purified water, sealing and placing same in a constant temperature oven, and keeping the temperature constant at 120°C for 2h; after cooling to room temperature, centrifuging same to take a certain volume of supernatant for the content test of copper ions. The results show that the dissolution rate of copper in water is 0.061%, or 0.054%.

### Example 21

### Preparation of carbon microbead loaded with zinc

Mixing zinc ions, small organic molecules and water for reaction to obtain a complex, and the specific operation is as follows:
taking 2 ml of 1.0 g/L zinc nitrate aqueous solution and adding it into a reaction flask, then adding a certain volume of 1.0 g/L 8-hydroxyquinoline-5-sulfonic acid solution, such that the molar weight of 8-hydroxyquinoline-5-sulfonic acid is 6 times of that of ferric ions, adjusting the pH value of the solution to 6.5, shaking same well to obtain a complex for later use;
adsorbing the complex with a carbon microbead, and the specific operation is as follows:
   weighing 1.0 g of the carbon microbead and adding same to the above solution, and stirring same for 10 minutes to complete the adsorption;
   performing a first treatment on the adsorbed carbon microbead, and the specific operation is as follows:
      taking the supernatant to measure the zinc content by the spectrophotometry method, and the calculated loading rate is 97.3%; taking 200 mg of the above carbon microbeads loaded with zinc, carrying out solid-liquid separation, placing same in a high-temperature furnace after drying, keeping the temperature constant at 800°C for 2 hours to obtain a carbon microbead loaded with zinc oxide.

Determination of the dissolution rate of zinc ions in the carbon microbead loaded with zinc: taking about 100 mg of the above carbon microbead products, and placing same in a hydrothermal reaction kettle, adding 10 ml of purified water, sealing and placing same in a constant temperature oven, and keeping the temperature constant at 120°C for 2h; after cooling to room temperature, centrifuging same to take a certain volume of supernatant for the content test of zinc ions. The results show that the dissolution rate of zinc in water is 0.021%.

### Example 22

### Preparation of carbon microbead loaded with titanium

Mixing titanium ions, small organic molecules and water for reaction to obtain a complex, and the specific operation is as follows:
taking 20 ml of 0.1 g/L titanium tetrachloride aqueous solution and adding it into a reaction flask, then adding a certain volume of 1.0 g/L 5-sulfosalicylic acid solution, such that the molar weight of 5-sulfosalicylic acid is 3 times of that of titanium ions, adjusting the pH value of the solution to 5.0, shaking same well to obtain a complex for later use;
adsorbing the complex with a carbon microbead, and the specific operation is as follows:
   weighing 1.0 g of the carbon microbead and adding same to the above solution, and stirring same for 10 minutes to complete the adsorption;
   performing a first treatment on the adsorbed carbon microbead, and the specific operation is as follows:
      taking the supernatant to measure the titanium content by the spectrophotometry method, and the calculated loading rate is 98.9%; taking 200 mg of the above carbon microbeads loaded with titanium, carrying out solid-liquid separation, placing same in a high-temperature furnace after drying, keeping the temperature constant at 800°C for 2 hours to obtain a carbon microbead loaded with titanium oxide.

Determination of the dissolution rate of titanium ions in the carbon microbead loaded with titanium: taking about 100 mg of the above carbon microbead products, and placing same in a hydrothermal reaction kettle, adding 10 ml of purified water, sealing and placing same in a constant temperature oven, and keeping the temperature constant at 120°C for 2 h; after cooling to room temperature, centrifuging same to take a certain volume of supernatant for the content test of titanium ions. The results show that the dissolution rate of titanium in water is 0.018%.

### Example 23

### Preparation of carbon microbead loaded with gallium

Mixing gallium ions, small organic molecules and water for reaction to obtain a complex, and the specific operation is as follows:
taking 2 ml of 0.1 g/L gallium nitrate aqueous solution and adding it into a reaction flask, then adding a certain volume of 1.0 g/L 2,6-pyridinedicarboxylic acid solution, such that the molar weight of 2,6-pyridinedicarboxylic acid is 4 times of that of gallium ions, adjusting the pH value of the solution to 6.0, shaking same well to obtain a complex for later use;
adsorbing the complex with a carbon microbead, and the specific operation is as follows:
   weighing 1.0 g of the carbon microbead and adding same to the above solution, and stirring same for 10 minutes to complete the adsorption;
   performing a first treatment on the adsorbed carbon microbead, and the specific operation is as follows:
      taking the supernatant to measure the gallium content using the spectrophotometry method, and the calculated loading rate is 97.9%; taking 200 mg of the above carbon microbeads loaded with gallium, carrying out solid-liquid separation, placing same in a high-temperature furnace after drying, keeping the temperature constant at 800°C for 2 hours to obtain a carbon microbead loaded with gallium oxide.

Determination of the dissolution rate of gallium ions in the carbon microbead loaded with gallium: taking about 100 mg of the above carbon microbead products, and placing same in a hydrothermal reaction kettle, adding 10 ml of purified water, sealing and placing it in a constant temperature oven, keeping the temperature constant at 120°C for 2h. After cooling to room temperature, centrifuging it to take a certain volume of supernatant for the content test of gallium ions. The results show that the dissolution rate of gallium in water is 0.088%.

## Claims

1. A method for preparing a carbon microbead loaded with a metal nuclide, **characterized in that** the method comprises the following steps:
- reacting metal ions and organic small molecules in an aqueous solution to obtain a complex, wherein the organic small molecules are selected from the group consisting of 5-sulfosalicylic acid, 5-nitrosalicylic acid, trimesic acid, phthalic acid, isophthalic acid, sodium catechol-3,5-disulfonate, pyrogallol, resorcinol, 8-hydroxyquinoline-5-sulfonic acid, 2,6-pyridinedicarboxylic acid, 2-pyridinecarboxylic acid, and 1,10-phenanthroline ; and the metal ions are selected from the group consisting of: scandium (Sc3+), yttrium (Y3+), lanthanum (La3+), cerium (Ce3+), praseodymium (Pr3+), neodymium (Nd3+), promethium (Pm3+), samarium (Sm3+), europium (Eu3+), gadolinium (Gd3+), terbium (Tb3+), dysprosium (Dy3+), holmium (Ho3+), erbium (Er3+), thulium (Tm3+), ytterbium (Yb3+), lutetium (Lu3+), copper (Cu2+), zinc (Zn2+), iron (Fe3+), cobalt (Co3+), nickel (Ni3+), titanium (Ti4+), gallium (Ga2+), and all non-radioactive and radioactive isotopes thereof.
- adsorbing the complex with a carbon microbead; and
- performing a first treatment on the adsorbed carbon microbead selected from any of the following:
i) mixing the adsorbed carbon microbead with an anion solution for reaction, wherein the anion solution is selected from the group consisting of phosphate, carbonate, tannin, and sulfide ion solutions;
ii) subjecting the adsorbed carbon microbead to an oxidation treatment wherein the metal ions adsorbed in the pore channels of the carbon microbead are oxidized into metal oxides; and
iii) subjecting the adsorbed carbon microbead to a reduction treatment, and reducing the metal ions adsorbed in the pore channels of the carbon microbead into metal particles; and
wherein the carbon microbead is a spherical or non-spherical activated carbon product rich in micropores and/or mesopores; the metal ions in the adsorbed carbon microbead are adsorbed in the pore channels of the carbon microbead.

2. The method of claim 1, **characterized in that** a mixed solution of the metal ions, the small organic molecules and water has a pH value of 1.0-12.0, preferably a pH value of 3.0-7.0; the molar ratio of the small organic molecules to the metal ions is 1-1000 : 1, preferably 1.5-15 : 1.

3. The method of claim 1, **characterized in that** the oxidation treatment is a high-temperature treatment in an inert gas atmosphere or an atmosphere containing trace oxygen, and the reduction treatment is a treatment with a reducing agent or a high-temperature reducing gas atmosphere.

4. A carbon microbead loaded with a metal nuclide prepared by the method of any one of claims 1-3, wherein the dissolution rates of the metal nuclides in the carbon microbeads loaded with metal nuclides in an aqueous solution at 0-180°C are lower than 0.1%.

5. Carbon microbead loaded with a metal nuclide according to claim 4 for its use in the treatment of solid liver tumors embolization with radioactive internal irradiation, and wherein the carbon microbead has a particle size of 0.1-1000 µm; and the carbon microbead loaded with a metal nuclide has an optimal particle size of 20-60 µm.

## Patentansprüche

1. Verfahren zur Herstellung einer mit einem Metallnuklid beladenen Kohlenstoffmikroperle, **dadurch gekennzeichnet, dass** das Verfahren die folgenden Schritte umfasst:
- Reagieren von Metallionen und organischen kleinen Molekülen in einer wässrigen Lösung, um einen Komplex zu erhalten, wobei die organischen kleinen Moleküle aus der Gruppe ausgewählt sind, die aus 5-Sulfosalicylsäure, 5-Nitrosalicylsäure, Trimesinsäure, Phthalsäure, Isophthalsäure, Natriumcatechol-3,5-disulfonat, Pyrogallol, Resorcin, 8-Hydroxychinolin-5-sulfonsäure, 2,6-Pyridindicarboxylsäure, 2-Pyridinicarboxylsäure und 1,10-Phenanthrolin besteht; und die Metallionen aus der Gruppe ausgewählt sind, die aus Folgendem besteht: Scandium (Sc3+), Yttrium (Y3+), Lanthan (La3+), Cerium (Ce3+), Praseodymium (Pr3+), Neodym (Nd3+), Promethium (Pm3+), Samarium (Sm3+), Europium (Eu3+), Gadolinium (Gd3+), Terbium (Tb3+), Dysprosium (Dy3+), Holmium (Ho3+), Erbium (Er3+), Thulium (Tm3+), Ytterbium (Yb3+), Lutetium (Lu3+), Kupfer (Cu2+), Zink (Zn2+), Eisen (Fe3+), Kobalt (Co3+), Nickel (Ni3+), Titan (Ti4+), Gallium (Ga2+) und allen nicht radioaktiven und radioaktiven Isotopen davon.
- Adsorbieren des Komplexes mit einer Kohlenstoffmikroperle; und
- Durchführen einer ersten Behandlung der adsorbierten Kohlenstoffmikroperle, ausgewählt aus einem von Folgendem:
i) Mischen der adsorbierten Kohlenstoffmikroperle mit einer Anionenlösung zur Reaktion, wobei die Anionenlösung aus der Gruppe ausgewählt wird, die aus Phosphat-, Karbonat-, Tannin- und Sulfidionenlösungen besteht;
ii) Unterziehen der adsorbierten Kohlenstoffmikroperle einer Oxidationsbehandlung, wobei die in den Porenkanälen der Kohlenstoffmikroperle adsorbierten Metallionen zu Metalloxiden oxidiert werden; und
iii) Unterziehen der adsorbierten Kohlenstoffmikroperle einer Reduktionsbehandlung und Reduzieren der in den Porenkanälen der Kohlenstoffmikroperle adsorbierten Metallionen in Metallpartikel; und
wobei die Kohlenstoffmikroperle ein kugelförmiges oder nichtkugelförmiges Aktivkohleprodukt ist, das reich an Mikroporen und/oder Mesoporen ist; die Metallionen in der adsorbierten Kohlenstoffmikroperle werden in den Porenkanälen der Kohlenstoffmikroperle adsorbiert.

2. Verfahren nach Anspruch 1, **dadurch gekennzeichnet, dass** eine gemischte Lösung der Metallionen, der kleinen organischen Moleküle und des Wassers einen pH-Wert von 1,0-12,0, vorzugsweise einen pH-Wert von 3,0-7,0 aufweist; das molare Verhältnis der kleinen organischen Moleküle zu den Metallionen beträgt 1-1,000: 1, vorzugsweise 1,5-1,5: 1.

3. Verfahren nach Anspruch 1, **dadurch gekennzeichnet, dass** die Oxidationsbehandlung eine Hochtemperaturbehandlung in einer Inertgasatmosphäre oder einer Atmosphäre ist, die Spurensauerstoff enthält, und die Reduktionsbehandlung eine Behandlung mit einem Reduktionsmittel oder einer Hochtemperatur-Reduktionsgasatmosphäre ist.

4. Kohlenstoffmikroperle, die mit einem Metallnuklid beladen und nach einem der Ansprüche 1 bis 3 hergestellt ist, wobei die Auflösungsraten der Metallnuklide in den mit Metallnukliden beladenen Kohlenstoffmikroperlen in einer wässrigen Lösung bei 0-180 °C geringer als 0,1 % sind.

5. Kohlenstoffmikroperle, die mit einem Metallnuklid beladen ist, nach Anspruch 4 zur Verwendung bei der Behandlung von soliden Lebertumoren durch Embolisation mit radioaktiver innerer Bestrahlung, und wobei die Kohlenstoffmikroperle eine Partikelgröße von 0,1-1000 µm aufweist; und die mit einem Metallnuklid beladene Kohlenstoffmikroperle eine optimale Partikelgröße von 20-60 µm aufweist.

## Revendications

1. Procédé de préparation d'une microbille de carbone chargée d'un nucléide métallique, **caractérisé en ce que** le procédé comporte les étapes suivantes :
- la mise en réaction d'ions métalliques et de petites molécules organiques dans une solution aqueuse pour obtenir un complexe, dans lequel les petites molécules organiques sont sélectionnées parmi le groupe consistant en l'acide 5-sulfosalicylique, l'acide 5-nitrosalicylique, l'acide trimésique, l'acide phtalique, l'acide isophtalique, le catéchol-3,5-disulfonate de sodium, le pyrogallol, le résorcinol, l'acide 8-hydroxyquinoline-5-sulfonique, l'acide 2,6-pyridinedicarboxylique, l'acide 2-pyridinecarboxylique et la 1,10-phénanthroline ; et les ions métalliques sont sélectionnés parmi le groupe consistant en : le scandium (Sc3+), l'yttrium (Y3+), le lanthane (La3+), le cérium (Ce3+), le praséodyme (Pr3+), le néodyme (Nd3+), le prométhium (Pm3+), le samarium (Sm3+), l'europium (Eu3+), le gadolinium (Gd3+), le terbium (Tb3+), le dysprosium (Dy3+), l'holmium (Ho3+), l'erbium (Er3+), le thulium (Tm3+), l'ytterbium (Yb3+), le lutétium (Lu3+), le cuivre (Cu2+), le zinc (Zn2+), le fer (Fe3+), le cobalt (Co3+), le nickel (Ni3+), le titane (Ti4+), le gallium (Ga2+), et tous les isotopes non radioactifs et radioactifs de ceux-ci.
- l'adsorption du complexe avec une microbille de carbone ; et
- la réalisation d'un premier traitement sur la microbille de carbone adsorbée sélectionné parmi l'un quelconque de ce qui suit :
i) le mélange de la microbille de carbone adsorbée avec une solution anionique pour une réaction, dans lequel la solution anionique est sélectionnée parmi le groupe consistant en des solutions ioniques de phosphate, de carbonate, de tanins et de sulfure ;
ii) la soumission de la microbille de carbone adsorbée à un traitement d'oxydation dans lequel les ions métalliques adsorbés dans les canaux de pores de la microbille de carbone sont oxydés en oxydes métalliques ; et
iii) la soumission de la microbille de carbone adsorbée à un traitement de réduction, et la réduction des ions métalliques adsorbés dans les canaux de pores de la microbille de carbone en particules métalliques ; et
dans lequel la microbille de carbone est un produit de charbon actif sphérique ou non sphérique riche en micropores et/ou mésopores ; les ions métalliques dans la microbille de carbone adsorbée sont adsorbés dans les canaux de pores de la microbille de carbone.

2. Procédé selon la revendication 1, **caractérisé en ce qu'**une solution mélangée des ions métalliques, des petites molécules organiques et d'eau possède une valeur de pH de 1,0 à 12,0, de préférence une valeur de pH de 3,0 à 7,0 ; le rapport molaire des petites molécules organiques sur les ions métalliques est de 1 à 1000: 1, de préférence de 1,5 à 15: 1.

3. Procédé selon la revendication 1, **caractérisé en ce que** le traitement d'oxydation est un traitement à haute température dans une atmosphère de gaz inerte ou une atmosphère contenant de l'oxygène à l'état de traces, et le traitement de réduction est un traitement avec un agent réducteur ou une atmosphère de gaz réducteur à haute température.

4. Microbille de carbone chargée d'un nucléide métallique préparée par le procédé selon l'une quelconque des revendications 1 à 3, dans laquelle les taux de dissolution des nucléides métalliques dans les microbilles de carbone chargées de nucléides métalliques dans une solution aqueuse à 0 à 180 °C sont inférieurs à 0,1 %.

5. Microbille de carbone chargée d'un nucléide métallique selon la revendication 4 pour son utilisation dans le traitement de l'embolisation de tumeurs hépatiques solides par irradiation radioactive interne, et dans laquelle la microbille de carbone possède une taille particulaire de 0,1 à 1000 µm ; et la microbille de carbone chargée d'un nucléide métallique possède une taille particulaire optimale de 20 à 60 µm.
